# EUROPEAN PATENT APPLICATION

(11) **EP 4 563 830 A1**
(43) Date of publication of application: **04.06.2025**
(21) Application number: 23846264.2
(22) Date of filing: 14.07.2023
(51) Int. Cl.: F16B 23/00, F16B 35/00, A61B 17/70, A61B 17/86

(54) **BONE SCREW**

(30) Priority: 29.07.2022 JP 2022121944
(71) Applicant: Spine-Tec Inc, Tokyo 105-0003 (JP); Takai Corporation, Mino-Shi, Gifu 501-3712 (JP)
(72) Inventor: MORITA, Kohei, Tokyo 105-0003 (JP); OHASHI, Hiroki, Tokyo 105-0003 (JP); TANI, Satoshi, Tokyo 105-0003 (JP); MURAYAMA, Yuichi, Tokyo 105-8461 (JP); TSUCHIDA, Kenji, Mino-shi, Gifu 501-3712 (JP); FURUTA, Yasuhiro, Mino-shi, Gifu 501-3712 (JP)
(74) Representative: SSM Sandmair
(86) International application number: PCT/JP2023/025979
(87) International publication number: WO 2024/024533

(57) **Abstract**

It is possible to provide a bone screw with sufficient strength. The bone screw of the present invention includes reinforced fibers and comprises a shaft, a tip located at the end of the shaft in the insertion direction, a head located at the end of the shaft in the removal direction, and a thread spirally located in the axial direction around the outer circumference of the shaft. The reinforced fibers are arranged along the axial direction of the shaft.

## Description

### TECHNICAL FIELD

The present invention relates to a bone screw that can be fixed to the bone of an animal.

### BACKGROUND OF INVENTION

In surgeries in fields such as neurosurgery, bone screws that include a tip for penetrating the bone have been developed to fix the bones of animals (especially humans) (see Patent Document 1).

Traditionally, bone screws made almost entirely of metal (such as stainless steel) or PEEK (registered trademark) have been proposed to maintain the strength required for bone fixation.

### CITATION LIST

### PATENT LITERATURE

Patent Document 1: Japanese Patent No. 5443398

### SUMMARY

Bone screws made almost entirely of metal (such as stainless steel) can cause artifacts when medical images are taken, which can interfere with surgery and other procedures that rely on medical imaging. Additionally, PEEK (registered trademark) may not always have sufficient strength.

The bone screw of the present invention includes reinforced fibers and comprises a shaft, a tip located at the end of the shaft in the insertion direction, a head located at the end of the shaft in the removal direction, and a thread spirally located in the axial direction around the outer circumference of the shaft. The reinforced fibers are arranged along the axial direction of the shaft.

According to the present invention, it is possible to provide a bone screw with sufficient strength.

### BRIEF DESCRIPTION OF THE DRAWINGS

Figure 1: Side view and cross-sectional view of the bone screw in this embodiment.
Figure 2: Cross-sectional view of the shaft and thread of the bone screw in this embodiment.
Figure 3: Diagram showing that the angle of the thread increases in the insertion direction of the bone screw, ranging from 60 to 80 degrees.
Figure 4: (a) Diagram showing the head of the bone screw in this embodiment. (b) Cross-sectional view along line B-B of the bone screw in this embodiment.
Figure 5: Perspective view of the bone screw in this embodiment.
Figure 6: Transparent view of the bone screw in this embodiment.
Figure 7: Cross-sectional view along line C-C of the bone screw in this embodiment.
Figure 8: Example of the type of thread used in the bone screw in this embodiment.
Figure 9: Another example of the type of thread used in the bone screw in this embodiment.
Figure 10: Diagram showing the bone screw housed in a housing and fixed with a nut, connected by a rod.
Figure 11: CT image (medical image) of the bone screw inserted into the bone of an animal.
Figure 12: Table evaluating the artifact of the bone screw in the medical image depending on the tantalum content.

### DESCRIPTION OF EMBODIMENTS

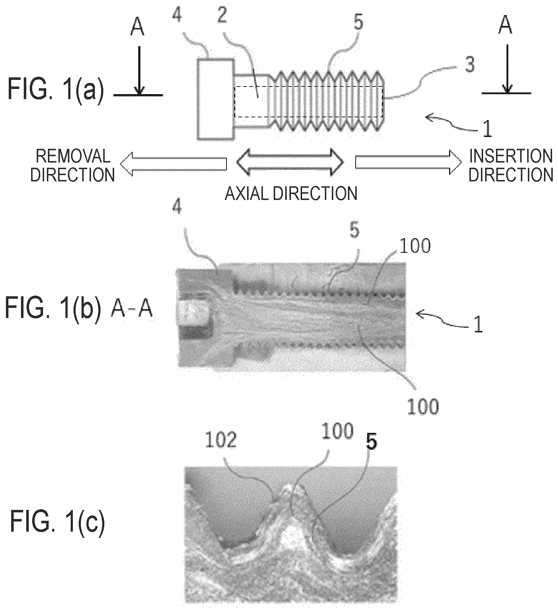

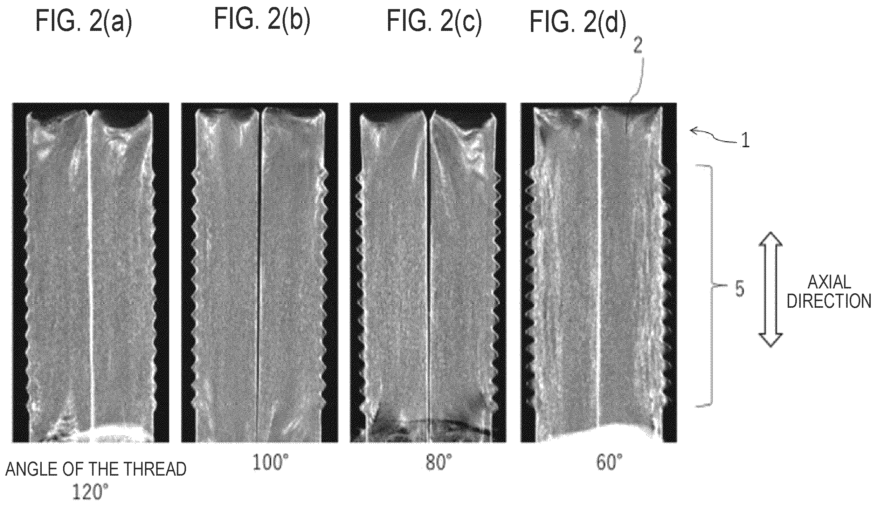

The bone screw of this embodiment will be explained with reference to the drawings. Figure 1 shows a side view and a cross-sectional view of the bone screw. Figure 2 shows a cross-sectional view of the shaft and thread of the bone screw.

As shown in Figure 1(a), the bone screw 1 of this embodiment includes reinforced fibers, a shaft 2, a tip 3 located at the end of the shaft in the insertion direction, a head 4 located at the end of the shaft in the removal direction, and a thread 5 that spirals around the outer circumference of the shaft 2 in the axial direction.

Figure 1(b) is a cross-sectional view along line A-A of Figure 1(a). As shown in Figure 1(b), the reinforced fiber 100 is arranged in a position that extends in the axial direction of the shaft 2. Figure 1(c) is an enlarged cross-sectional view of the thread 5 from Figure 1(b). As shown in Figure 1(c), the reinforced fiber 100 bends within the thread 5.

Additionally, though not shown in the figures, the reinforced fiber 100 is arranged in the same direction as the spiral of the thread 5. The reinforced fiber 100 extends along the spiral direction of the thread 5.

Furthermore, although not shown, the reinforced fiber 100 can also be wound in a helical shape around at least one of the shaft 2 or the thread 5.

When molding the bone screw using the reinforced fiber 100, a composite material made by mixing the reinforced fiber 100 (e.g., carbon fiber of 1mm) and a resin (e.g., vinyl ester) is injected into the mold of the bone screw from the head side of the screw towards the insertion direction. In this case, when composing the mixture, the reinforced fiber 100 is mixed with the resin in such a way that the fiber 100 is arranged in a predetermined direction. For example, the reinforced fiber 100 can be mixed so that it is arranged in the axial direction of the bone screw 1, or it can be arranged at a specific angle to the axial direction. Alternatively, the fiber 100 can be arranged along the spiral direction of the thread 5. The resin includes at least one of thermosetting resins and thermoplastic resins. For example, resins can be selected from epoxy resins, phenolic resins, unsaturated polyester resins, and others.

By injecting this composite material into the mold, the reinforced fiber 100 is positioned correctly in the shaft 2, extending in the axial direction, or bending in the thread 5, or extending along the spiral direction of the thread 5, or wound in a helical shape around at least one of the shaft 2 or the thread 5.

In the molded bone screw 1, the reinforced fiber 100 is appropriately positioned in the specified direction, which enhances at least one of the compressive strength, tensile strength, shear strength, torsional strength, and hardness of the bone screw 1.

The reinforced fiber 100 preferably includes at least one of carbon fiber, aramid fiber, or glass fiber, but is not limited to these.

Figure 2 is a cross-sectional view of a bone screw 1 that has been molded using the composite material of reinforced fiber 100 and resin. The angle of the thread 5 is between 60 and 80 degrees.

The inventors conducted diligent research and discovered the optimal angle of the thread 5 to properly mold the thread 5 of the bone screw 1 by injecting the composite material of reinforced fiber 100 and resin into the mold. If the angle of the thread portion in the mold is too small, the composite material will not be adequately injected into the thread portion, resulting in an improperly molded thread 5. Conversely, if the angle of the thread portion is too large, the fixation strength of the bone screw 1 to the bone will be insufficient, and it will easily detach from the bone.

Figure 2(a) and Figure 2(b) show examples where the angle of the thread 5 is 120 degrees and 100 degrees, respectively. As shown in these figures, although the composite material is adequately injected into the thread portion, the thread 5 with too large an angle can easily detach from the bone.

Figure 2(c) and Figure 2(d) show examples where the angle of the thread 5 is 80 degrees and 60 degrees, respectively. As shown in these figures, the composite material is adequately injected into the thread portion, and the thread 5 does not easily detach from the bone. Therefore, it is appropriate for the angle of the thread 5 to be between 60 and 80 degrees. Preferably, the angle of the thread 5 is between 60 and 75 degrees, and more preferably between 65 and 70 degrees.

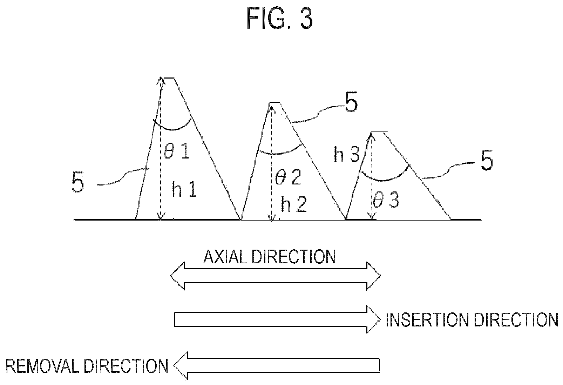

Figure 3 is a diagram showing that the angle of the thread 5 increases in the insertion direction of the bone screw 1, ranging from 60 to 80 degrees. As shown in Figure 3, the angles *θ*1 to *θ*3 of the thread 5 increase in the insertion direction of the bone screw 1, with *θ*1 < *θ*2 *< θ*3, all within the range of 60 to 80 degrees. Preferably, the angle of the thread 5 increases within the range of 60 to 75 degrees in the insertion direction of the bone screw 1, and more preferably within the range of 65 to 70 degrees.

The angle of the thread 5 may increase progressively or intermittently within the above range in the insertion direction of the bone screw 1. Additionally, the height h1 to h3 of the thread 5 may decrease in the insertion direction. The height of the thread 5 may change according to the angle of the thread 5.

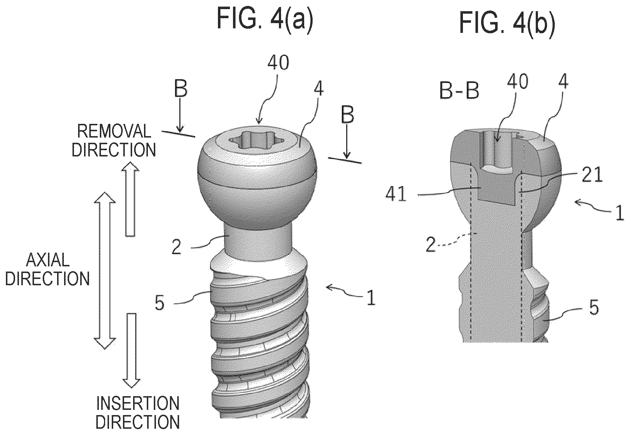

Figure 4(a) shows the head 4 of the bone screw 1. Figure 4(b) is a cross-sectional view along line B-B of the bone screw 1. As shown in Figure 4(b), the head 4 has a head projection 41 in the insertion direction, and the head projection 41 fits into the shaft recess 21 of the shaft 2. Furthermore, the connection point (the portion) between the head 4 and the shaft 2 (the head projection 41) has at least one of higher compressive strength, tensile strength, shear strength, torsional strength, or hardness compared to the shaft 2. For example, the head projection 41 includes at least one of metal (such as titanium, stainless steel, iron, nickel, cobalt, or cemented carbide), ceramic (such as zirconia), cermet, and reinforced fibers of a different composition from the reinforced fiber 100.

Here, "reinforced fibers of a different composition" refers to reinforced fibers with a different mixing ratio, length, shape, or arrangement than the reinforced fiber 100. Even if the same type of reinforced fiber is used, it is acceptable as long as it has different characteristics from reinforced fiber 100. Alternatively, if other reinforced fibers are mixed, the same type of reinforced fiber can be used as long as it is of a different composition from the reinforced fiber 100.

Without relying on the head projection 41 or the shaft recess 21, the connection point (the portion) between the head 4 and the shaft 2 should have at least one of higher compressive strength, tensile strength, shear strength, torsional strength, or hardness compared to the shaft 2.

For example, although not shown, the shaft 2 may include a shaft projection in the removal direction, and the shaft projection may fit into a head recess of the head 4. In this case, the head 4 has a recess, and the shaft 2 has a projection, but it can be reversed, with the shaft 2 having a projection and the head 4 having a recess. Moreover, the core part may include at least one of metal (such as titanium, stainless steel, iron, nickel, cobalt, or cemented carbide), ceramic (such as zirconia), cermet, and reinforced fibers of a different composition from the reinforced fiber 100, and the core part may fit into the head recess of the head 4 and the shaft recess of the shaft 2.

That is, the head 4 and the shaft 2 are provided with recesses, and these recesses are connected by a core part. In addition, the connection point between the head 4 and the shaft 2 should have at least one area with higher compressive strength, tensile strength, shear strength, torsional strength, or hardness compared to the shaft 2. In this case, the boundary surface between the head 4 and the shaft 2 may consist of different materials, as shown in Figure 4, or it may consist of the same material (e.g., the same type of reinforced fiber) without a clear boundary surface. For example, even if there is no clear boundary surface, the connection point can have at least one area with higher compressive strength, tensile strength, shear strength, torsional strength, or hardness, if the mixing ratio, length, shape, or arrangement of the same material (e.g., reinforced fiber) is continuously different.

By reinforcing at least one area between the head 4 and the shaft 2 that is subject to load, it is possible to improve the compressive strength, tensile strength, shear strength, torsional strength, or hardness and prevent the bone screw 1 from breaking or deforming.

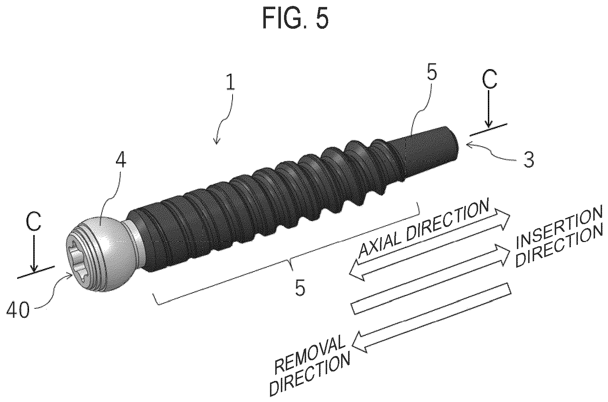

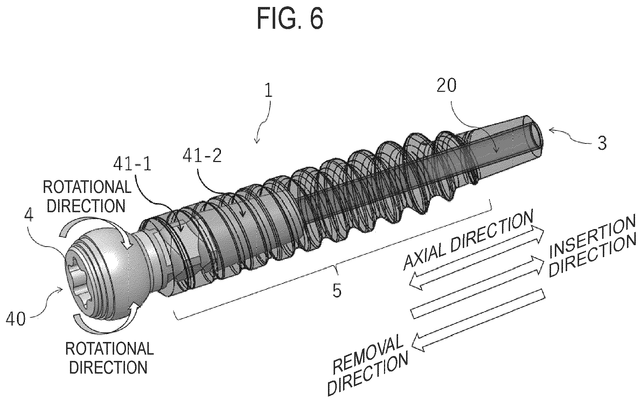

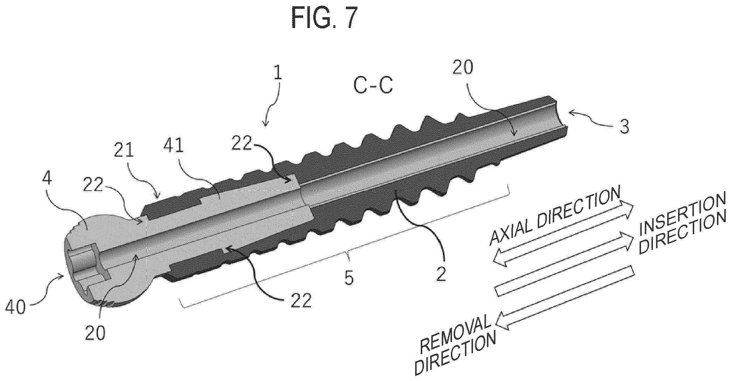

Figure 5 is a perspective view of the bone screw 1, Figure 6 is a transparent view of the bone screw 1, and Figure 7 is a cross-sectional view along line C-C of the bone screw 1.

As shown in Figures 5 to 7, the head 4 has a head projection 41 in the insertion direction, and the head projection 41 fits into the shaft recess 21 of the shaft 2.

As shown in Figure 6, the head projection 41 includes a polygonal section 41-1 that is perpendicular (oriented perpendicularly) to the axial direction. The polygonal section 41-1 is approximately rectangular in cross-section and chamfered. Preferably, the polygonal section 41-1 is approximately triangular to hexagonal. By making the cross-section of the head projection 41 polygonal and perpendicular to the axial direction, if torque is applied to the bone screw 1 in the rotational direction, the head projection 41 can prevent slipping or detachment from the shaft recess 21 in the rotational direction.

As shown in Figure 7, the head projection 41 includes a notch 22. The notch 22 may be a rotation surface in the rotational direction of the bone screw 1 or may have a flange shape. By providing the head projection 41 with a notch 22, it can prevent the head projection 41 from slipping or detaching from the shaft recess 21 in the axial direction. Furthermore, though not shown, the head projection 41 may also include threads that are in the reverse spiral direction to the threads 5 of the bone screw.

Similarly, the shaft recess 21 may include at least one of a polygonal section or threads located in the reverse spiral direction to the threads 5, and a notch.

In cases where the shaft projection of the shaft 2 fits into the recess of the head 4 or where a core part fits into the head recess of the head 4 and the shaft recess of the shaft 2, the projection of the shaft 2, the recess of the head 4, and the core part may include at least one of a polygonal section or threads located in the reverse spiral direction to the threads 5, and a notch.

The core part may include reinforced fibers arranged in at least one of a position extending in the axial direction and a position spirally wound around the axial direction.

As shown in Figures 6 and 7, the bone screw 1 includes a hole 20 that penetrates the interior of the bone screw 1 in the axial direction. In Figures 6 and 7, the hole 20 extends from the recess 40 of the head 4 to the tip 3, penetrating the interior of the bone screw 1.

The hole 20 is surrounded by a material that includes at least one of metal (such as titanium, stainless steel, iron, nickel, cobalt, or cemented carbide), ceramic (such as zirconia), cermet, and reinforced fibers of a different composition from reinforced fiber 100. Here, "reinforced fibers of a different composition" refers to reinforced fibers with a different mixing ratio, length, shape, or arrangement compared to reinforced fiber 100. The same type of reinforced fiber can be used as long as its composition differs from that of reinforced fiber 100. Additionally, if other reinforced fibers are mixed, the same type of reinforced fiber can be used as long as it has a different composition.

When the hole 20, surrounded by a material containing reinforced fibers of a different composition from fiber 100, appears brighter than the surrounding area in a medical image such as a CT scan, the hole 20 serves as a guide for identifying the position and direction of the bone screw 1 in medical images.

At least a part of the hole 20 should be surrounded by a material that includes at least one of metal (such as titanium, stainless steel, iron, nickel, cobalt, or cemented carbide), ceramic (such as zirconia), cermet, and reinforced fibers of a different composition from reinforced fiber 100.

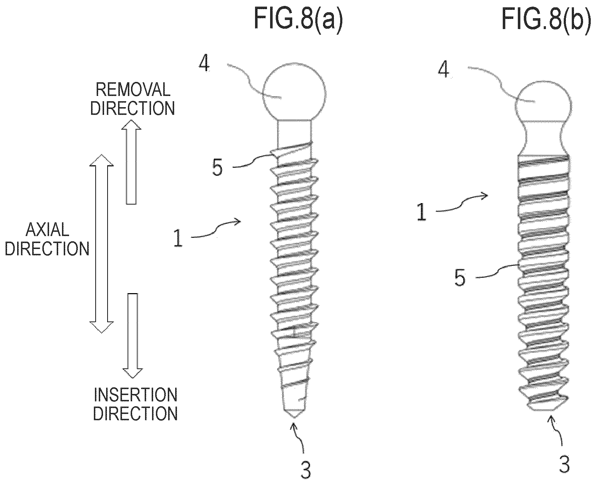

Figure 8 shows examples of thread types for the bone screw 1. In the bone screw 1 shown in Figure 8(a), the flank angle on the insertion side of the thread 5 is larger than the flank angle on the removal side. In the bone screw 1 shown in Figure 8(b), the shaft 2 narrows towards the insertion direction, and the diameter of the root of the thread 5 becomes smaller towards the insertion direction. Meanwhile, the outer diameter of the thread 5 remains roughly constant. In the bone screw 1 shown in Figure 8(b), the height of the thread 5 increases in the insertion direction. Additionally, in the bone screw 1 shown in Figure 8(b), the axial length of the crest of the thread 5 decreases in the insertion direction.

Such a thread shape enables the bone screw 1 to be securely fixed to the bone while also allowing for easy removal from the bone.

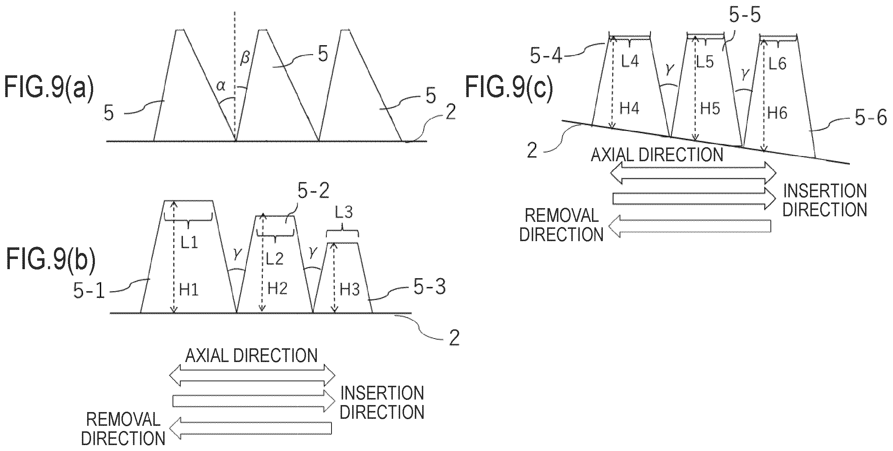

Figure 9 shows other examples of thread types for the bone screw 1. Figure 9 is a cross-sectional view of the threads 5 of the bone screw 1. In the bone screw 1 shown in Figure 9(a), the flank angle α on the insertion side of the thread 5 is larger than the flank angle *β* on the removal side. In the bone screw 1 shown in Figure 9(b), the heights H1, H2, H3 of the threads 5-1, 5-2, 5-3 increase in the insertion direction. In the bone screw 1 shown in Figure 9(b), the axial lengths L1, L2, L3 of the crests of the threads 5-1, 5-2, 5-3 decrease in the insertion direction. The diameter of the root of the thread 5 and the angle *γ* of the thread 5 remain roughly constant. In the bone screw 1 shown in Figure 9(c), the shaft 2 narrows towards the insertion direction, and the diameter of the root of the thread 5-4, 5-5, 5-6 becomes smaller in the insertion direction. Meanwhile, the outer diameter of the thread 5 and the angle *γ* of the thread 5 remain roughly constant. The heights H4, H5, H6 of the threads 5-4, 5-5, 5-6 increase in the insertion direction. In the bone screw 1 shown in Figure 9(c), the axial lengths L4, L5, L6 of the crests of the threads 5-4, 5-5, 5-6 decrease in the insertion direction.

Such thread shapes enable the bone screw 1 to be securely fixed to the bone while also allowing for easy removal from the bone.

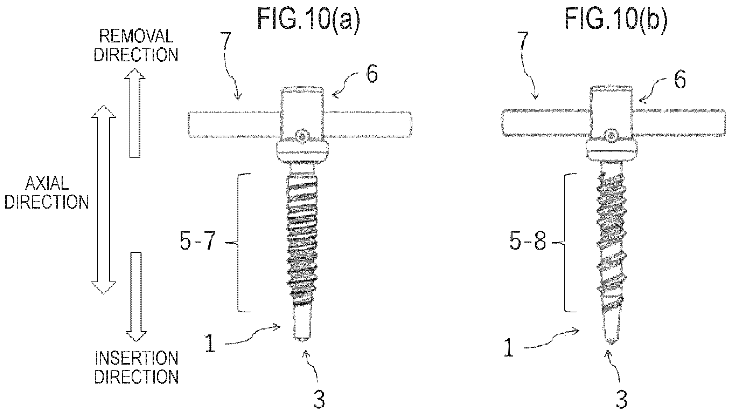

Figure 10 shows a bone screw 1 housed in a housing 6, fixed inside the housing 6 by a nut (not shown), and connected by a rod 7.

As shown in Figure 10(a), the thread 5-7 of the bone screw 1 has a similar shape to the thread 5 in Figure 8(b). As shown in Figure 10(b), the pitch of the thread 5 increases in the insertion direction.

Such a thread shape allows the bone screw 1 to be securely fixed to the bone while also enabling easy removal.

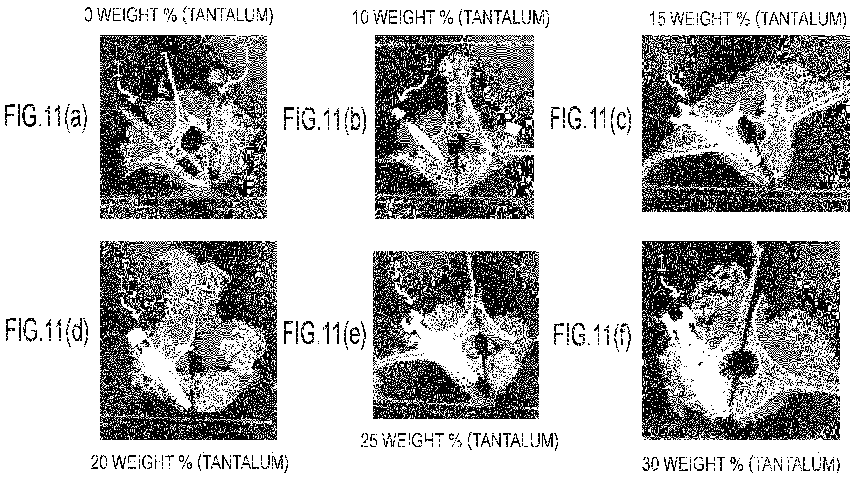

Figure 11 is a CT image (medical image) of the bone screw 1 inserted into the bone of an animal (pig). In Figure 11, the bone screw 1 contains a composite mixture in which tantalum, at a concentration of more than 0% but less than or equal to 30% (0%, 10%, 15%, 20%, 25%, 30% by weight), is mixed into a composition containing reinforced fibers.

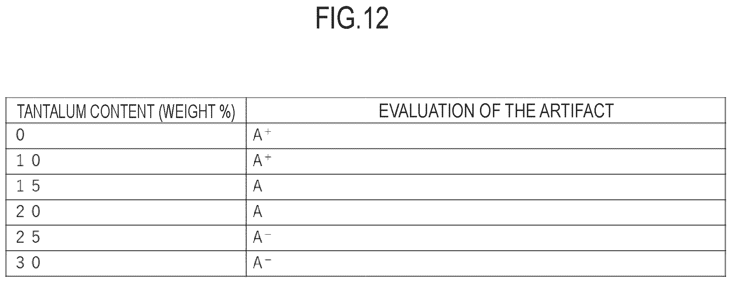

Figure 12 is a table evaluating the artifact of the bone screw 1 in the medical image depending on the tantalum content, as shown in Figure 11.

As shown in Figures 11 and 12, the higher the tantalum content in the mixture, the stronger the artifact of the bone screw 1 in the CT image (medical image). When the tantalum content in the mixture is 25% or 30% by weight, the artifact evaluation is rated A-, which is acceptable. It is predicted that similar results will occur with other metals (such as titanium, stainless steel, iron, nickel, cobalt, and cemented carbide). Therefore, the bone screw 1 (including at least one of the shaft 2, tip 3, head 4, and thread 5) contains a composite mixture in which metal (such as tantalum, titanium, stainless steel, iron, nickel, cobalt, and cemented carbide) is mixed into a composition containing reinforced fibers at a concentration of more than 0% and less than or equal to 30% by weight (preferably between 10% and 20% by weight, and more preferably between 10% and 15% by weight).

While reducing the artifact, if at least one of the shaft 2, tip 3, head 4, or thread 5 of the bone screw 1 appears brighter than the surrounding area in a medical image, the bone screw 1 functions as a guide for identifying the position and direction of the bone screw 1 in the medical image.

Additionally, the tip 3 may include materials that produce higher brightness in medical images compared to the reinforced fibers 100. For example, the tip 3 may include at least one of metal (such as tantalum, titanium, stainless steel, iron, nickel, cobalt, and cemented carbide), ceramic (such as zirconia), cermet, and reinforced fibers of a different composition from the reinforced fiber 100.

If the tip 3, which includes such materials, appears brighter than the surrounding area in a medical image, such as a CT scan, it functions as a guide for identifying the position and direction of the bone screw 1 in the medical image.

The embodiments of the present invention have been described above, but the present invention is not limited to these, and various modifications and alterations can be made within the scope of the claims.

### INDUSTRIAL APPLICABILITY

The present invention is useful as a bone screw with sufficient strength.

### EXPLANATION OF SYMBOLS

1...Bone screw
2...Shaft
3...Tip
4...Head
5...Thread
6...Housing
7...Rod
20...Hole
21...Shaft recess
22...Notch
40...Recess
41...Head projection
41-1...Polygonal section
100...Reinforced fiber

## Claims

1. A bone screw including reinforced fiber, comprising:
a shaft,
a tip located at the end of the shaft in the insertion direction,
a head located at the end of the shaft in the removal direction, and
a thread spirally located in the axial direction around the outer circumference of the shaft,
wherein the reinforced fibers are arranged along the axial direction of the shaft.

2. The bone screw according to claim 1, further comprising:
a portion between the head and the shaft that has at least one of the following properties: higher compressive strength, higher tensile strength, higher shear strength, higher torsional strength, and higher hardness compared to the shaft.

3. The bone screw according to claim 1 or claim 2, **characterized in that** the head includes a head projection in the insertion direction, and the head projection fits into a shaft recess of the shaft.

4. The bone screw according to claim 3, **characterized in that** at least one of the head projection and the shaft recess has at least one of a polygonal cross-section perpendicular to the axial direction, a thread located in the reverse spiral direction of the screw thread, and a notch.

5. The bone screw according to claim 3, **characterized in that** the head projection includes at least one of metal, ceramic, cermet, and reinforced fibers of a composition different from the reinforced fibers.

6. The bone screw according to claim 1 or claim 2, **characterized in that** the shaft includes a shaft projection in the removal direction, and the shaft projection fits into a head recess of the head.

7. The bone screw according to claim 6, **characterized in that** at least one of the shaft projection and the head recess has at least one of a polygonal cross-section perpendicular to the axial direction, a thread located in the reverse spiral direction of the screw thread, or a notch.

8. The bone screw according to claim 1 or claim 2, further comprising:
a core that includes at least one of metal, ceramic, cermet, and reinforced fibers of a composition different from the reinforced fibers,
wherein the core fits into the head recess and the shaft recess.

9. The bone screw according to claim 8, **characterized in that** the core includes reinforced fibers arranged in at least one of a position extending in the axial direction and a position spirally wound around the axial direction.

10. The bone screw according to claim 1 or claim 2, **characterized in that** the bone screw includes a hole configured to penetrate the interior of the bone screw in the axial direction,
and the hole is surrounded by a material that includes at least one of metal, ceramic, cermet, and reinforced fibers of a composition different from the reinforced fibers.

11. The bone screw according to claim 1 or claim 2, **characterized in that** the tip includes a material that produces a higher brightness in medical imaging compared to the reinforced fibers.

12. The bone screw according to claim 1 or claim 2, **characterized in that** the reinforced fibers are arranged in a position spirally wound around the axial direction of at least one of the shaft and the thread.

13. The bone screw according to claim 1 or claim 2, **characterized in that** the reinforced fibers are arranged in the same direction as the spiral direction of the thread in the thread.

14. The bone screw according to claim 1 or claim 2, **characterized in that** the reinforced fibers are bent within the thread.

15. The bone screw according to claim 1 or claim 2, **characterized in that** the reinforced fibers include at least one of carbon fibers, aramid fibers, and glass fibers.

16. The bone screw according to claim 1 or claim 2, **characterized in that** at least one of the shaft, the tip, the head, and the thread includes a mixture of metal at more than 0% and less than or equal to 30% by weight, combined with a composition that includes reinforced fibers.

17. The bone screw according to claim 1 or claim 2, **characterized in that** the angle of the thread is between 60 degrees and 80 degrees.

18. The bone screw according to claim 1 or claim 2, **characterized in that** the angle of the thread increases in the insertion direction within a range of 60 degrees to 80 degrees.

19. The bone screw according to claim 1 or claim 2, **characterized in that** the flank angle of the thread in the insertion direction is larger than the flank angle of the thread in the removal direction.

20. The bone screw according to claim 1 or claim 2, **characterized in that** the height of the thread increases in the insertion direction.

21. The bone screw according to claim 1 or claim 2, **characterized in that** the axial length of the crest of the thread decreases in the insertion direction.

22. The bone screw according to claim 1 or claim 2, **characterized in that** the pitch of the thread increases in the insertion direction.
